# EUROPEAN PATENT APPLICATION

(11) **EP 1 394 258 A1**
(43) Date of publication of application: **03.03.2004**
(21) Application number: 02019453.6
(22) Date of filing: 30.08.2002
(51) Int. Cl.: C12N 15/86

(54) **Non-human herpesviruses as vectors**

(71) Applicant: Geneart GmbH, 93053 Regensburg (DE)
(72) Inventor: Beer, Martin, 17498 Neuenkirchen (DE); Osterrieder, Nikolaus, Ithaca, NY 14850 (US); Rudolph, Jens, 17498 Insel Riems (DE); Trapp, Sascha, 17498 Insel Riems (DE); Wagner, Ralf, 93049 Regensburg (DE)
(74) Representative: Dehmel, Albrecht, Dr.

(57) **Abstract**

The present invention relates to animal viruses such as animal herpesviruses as a vector for diagnostic, therapeutic and prophylactic delivery of foreign genes and nucleic acids to animals, human or primary cells derived thereof, respectively. By using said viruses, the prevention and treatment of infectious, autoimmune and tumor diseases as well as allergies and genetic disorders can be treated. The present invention relates further to procedures to efficiently transduce primary human cells with recombinant animal viruses *in vitro* and *in vivo*. The invention further relates to a kit useful (i) for diagnosis and monitoring of innate and adoptive immune responses as well as (ii) for gene therapy and (iii) immunization purposes.

## Description

The present invention relates to animal viruses such as animal herpesviruses as a vector for diagnostic, therapeutic and prophylactic delivery of foreign genes and nucleic acids to animals, human or primary cells derived thereof, respectively. By using said viruses, the prevention and treatment of infectious, autoimmune and tumor diseases as well as allergies and genetic disorders can be achieved. The present invention relates further to procedures to efficiently transduce primary human cells with recombinant animal viruses *in vitro* and *in vivo*.

Equine herpesvirus type 1 (EHV-1), a member of the *Alphaherpesvirinae* (EHV-1 is an *Alphaherpesvirus*), genus *Varicellovirus,* normally infects and replicates in equines and cells of equine or rodent origin. Usually, the disease induced by the virus is mild and approximately 90% of equines worldwide harbor the agent, which persists in animals live-long. The clinical symptoms are mild respiratory disease and very rarely abortions in mares and neurological disease ¹.

The entire DNA sequence of one EHV-1 strain, Ab4p, has been determined. EHV-1 is one of eight equine herpesviruses identified so far. EHV-1 and its closest relative, EHV-4 are members of the *Varicellovirus* genus, and belong as EHV-3 and EHV-5 to EHV-8 to the *Alphaherpesvirinae.* EHV-2 and EHV-5 are *Gammaherpesvirinae* and are widespread in equine populations¹. EHV-1, -4, -3, -5 and EHV-8 are considered to closely related both regarding their tissue specificity, genome organization and gene functions. EHV-2 and EHV-8, although belonging to the *Gammaherpesvirinae* share the overall genome organization and many details also in gene functions with EHV-1, -4, -3, -5 and EHV-8

Herpesviruses enter target cells by fusion of their envelope with the plasma membrane. Glycoproteins are crucially involved in these early stages of infection, but also in the egress of virions from infected cells, and in direct cell-to-cell spread *(ctcs).* To date, 11 glycoproteins (g) in herpes simplex virus type 1 (HSV-1), the prototype *Alphaherpesvirus,* have been identified, and have been designated gB, gC, gD, gE, gG, gH, gI, gJ, gK, gL, and gM³. Comparisons of nucleotide and corresponding amino acid sequences revealed that all known HSV-1 glycoprotein genes are conserved in EHV-1. EHV-1 glycoproteins are named in accordance with the nomenclature used for the HSV-1 glycoproteins, but EHV-1 encodes a further envelope protein, named gp2, and a tegument protein, the VP13/14 homolog of HSV-1, which has been reported to be glycosylated. Although *Alphaherpesvirinae* genetically are closely related to each other, express similar sets of genes, and enter cells by comparable mechanisms, the proteins involved in this process but also in egress and *ctcs* differ considerably. For example, gD is absolutely required for entry and *ctcs* of HSV-1, EHV-1 and bovine herpesvirus type 1 (BHV-1), whereas it is absent from the prototype member of the *Varicellovirus* genus, varicella zoster virus (VZV) ⁴. The principal steps of virus entry can be distinguished into (i) heparin-sensitive and -insensitive attachment and (ii) fusion of the virus envelope with the target cell plasma membrane. Heparin-sensitive attachment, i.e. primary attachment that is mediated by interaction of the virus envelope with glycosaminoglycans on the cell surface, is conferred by gC in a number of *Alphaherpesvirinae* like pseudorabies virus (PRV), HSV-1, and EHV-1. After conformational changes of probably both the viral envelope and the plasma membrane, stable attachment conferred by interaction of gD with cellular receptors ensues Following stable attachment, fusion of the viral with the host cell plasma membrane occurs. This step is mediated in the *Alphaherpesvirinae* including EHV-1 by a process involving at least gB, gD and the gH-gL complex. The exact mechanism of fusion and the formation of a fusion pore, however, still remains enigmatic and no 'fusion peptide' could be identified so far. The glycoproteins also appear to determine ― at least partially ― the restricted *in vivo* host tropism of *Alphaherpesvirinae,* i.e. the inability of animal herpesviruses to infect species other than their host with which they have co-evolved.

In recent years, manipulation of the large herpesvirus genomes has been facilitated by the introduction of bacterial artificial chromosome (BAC) cloning and mutagenesis. The genomes of several herpesviruses including EHV-1 have been cloned as infectious BACs using this technique (^{7,89}. Fast protocols for targeted and random mutagenesis of herpesvirus genomes cloned as BACs have been used and mutagenesis is no longer dependent on growth of the viruses in eukaryotic cells but can be performed in *Escherichia coli* ¹⁰.

Several vectors for potential use in human gene therapy or immunizations have been described. Among those are RNA and DNA viruses. The most commonly used vectors are retroviruses, rhabodviruses, adenoviruses, adeno-associated viruses (AAV), and human herpesviruses including HSV-1, Epstein-Barr virus (EBV), and human cytomegalovirus (HCMV). The problems associated with RNA viruses, adenoviruses or AAV is their limited capacity to package foreign DNA, which is normally restricted to DNA of < 5 kbp in length¹¹. In addition, extremely high virus titers of adeno- or AA viruses are necessary for efficient transduction of human cells, explaining the risk for allergic or toxic reactions in patients^{12,13}. Human herpesviruses can package DNA of > 100 kbp in size and the limitations caused by the low packaging capacity intrinsic to the RNA, adenovirus or AAV systems can be overcome by using HSV-1, EBV or HCMV. However, a major disadvantage for using human viruses is the fact that most patients have already encountered infection and/or vaccination with those viruses ^{14,15}.

Accordingly, the problem of the present invention is the provision of novel vector systems to enable successful gene therapy approaches and to establish novel and efficacious immunization schemes.

The problem of the invention is solved by the subject matter defined in the claims.

The invention may be more easily understood by reference to the accompanying figures.

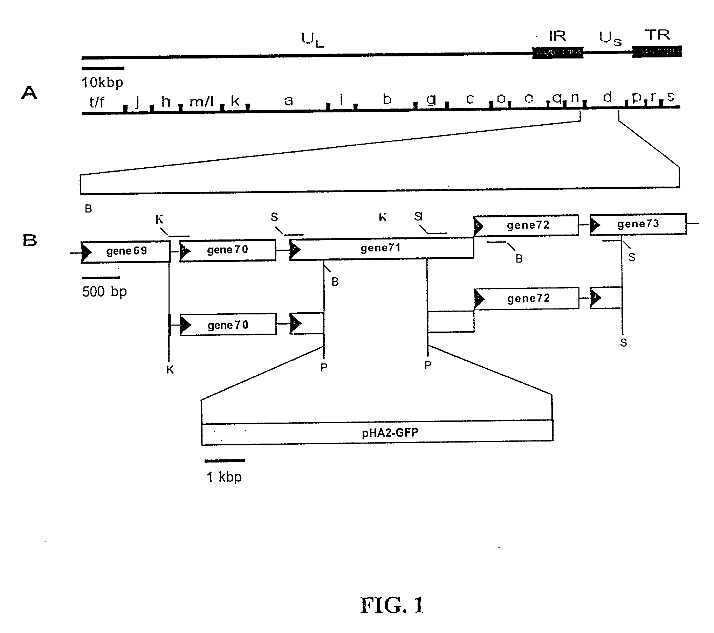

**Figure 1** schematically illustrates the construction of EHV-1 RacH BAC (pRacH). Shown is the organization of the approximately 150 kbp RacH genome (A). The genomic organization of the unique short region of the RacH genome with and without inserted BAC sequences is depicted (B). Scales in bp or kbp and restriction enzyme sites are given. B, *Bam*HI; H, *Hind*III; K, *Kpn*I; P, *Pac*I; S, *Sph*I; S1, *Sal*I.

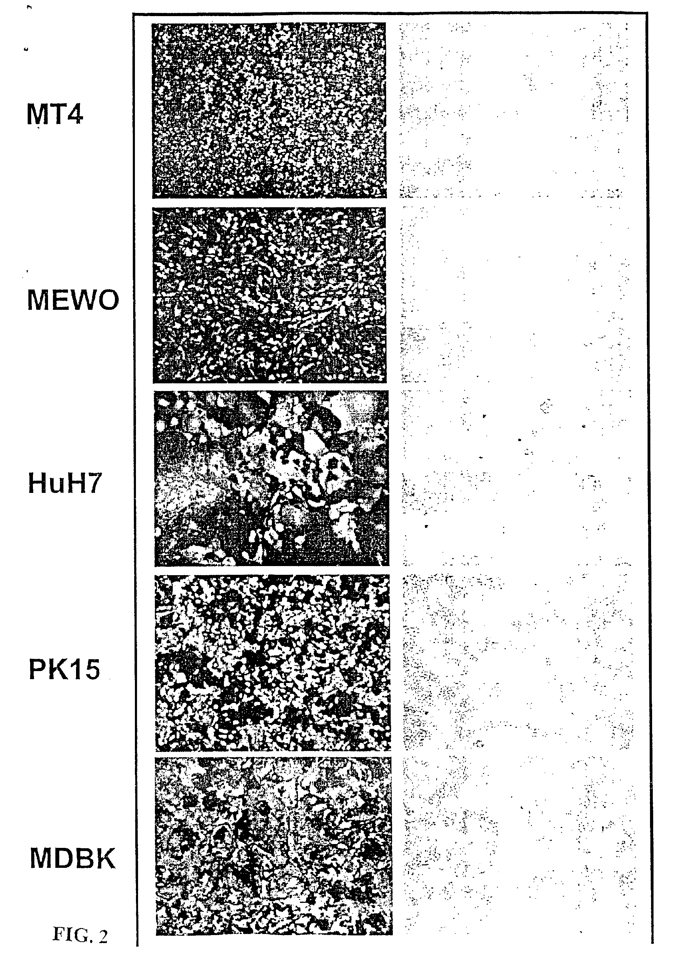

**Figure 2** shows the fluorescence analysis of human (MT4, MEWO, HuH7), porcine (PK15) and bovine (MDBK) cell lines after infection with HΔgp2 after addition of one infectious unit per cell. GFP expression was detected in more than 90% of the cells, independently of the cell type. Formation of syncytia was observed in HΔgp2-infected HuH7 and MDBK cells.

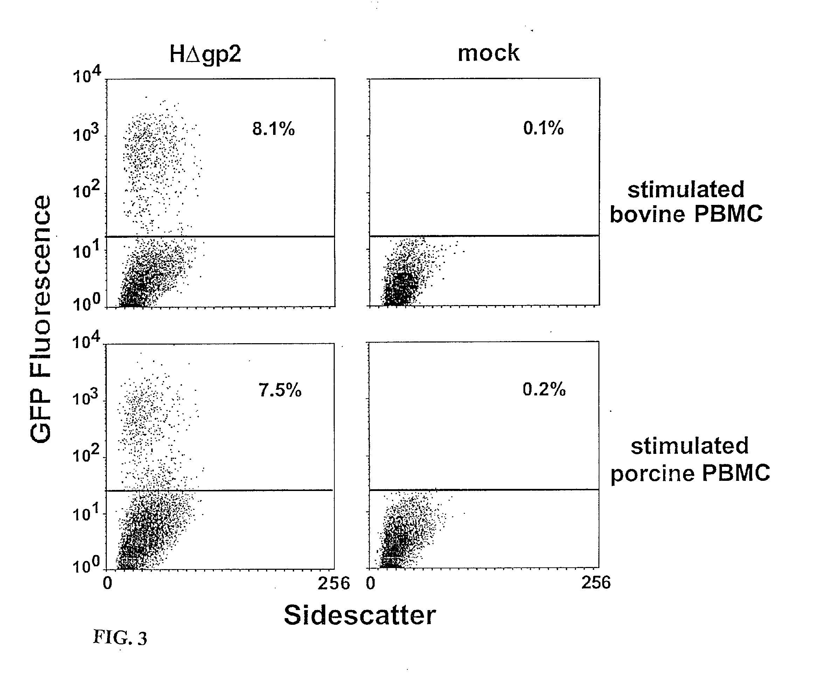

**Figure 3** shows flow cytometry data of transduced cells. Shown is a dotplot analysis of Con A-stimulated bovine and porcine PBMC 24h after inoculation (0.5 infectious units per cell) of EHV-1 HΔgp2. In comparison to mock-inoculated cells, expression of GFP could be detected in 8.1% and 7.5% of stimulated bovine and porcine PBMC, respectively.

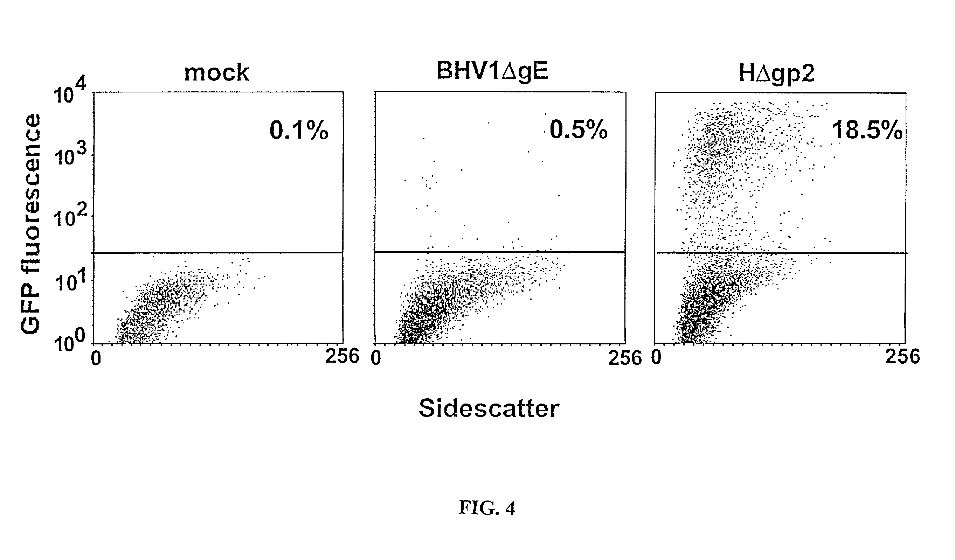

Figure 4 likewise shows flow cytometry data of transduced cells. Shown is a dot plot analysis of Con A-stimulated human PBMC at 24 hr after inoculation (0.5 infectious units per cell) of cells with EHV-1 HΔgp2 or BHV-1ΔgE. While 18.5% of the cells were positive for GFP expression after transduction using HΔgp2, less than 0.5% of the BHV-1ΔgE-inoculated PBMC exhibited a green fluorescence.

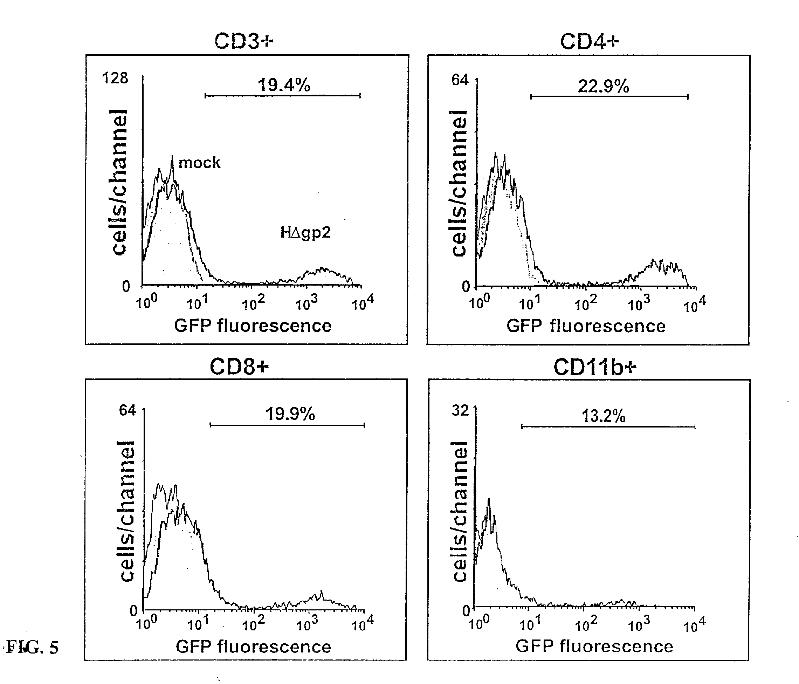

**Figure 5** shows a flow cytometric analysis of Con A-stimulated human PBMC at 24 hr after inoculation (0.5 infectious units per cell) of EHV-1 HΔgp2. Following inoculation, cells were stained by indirect immunofluorescence using CD3-, CD4-, CD8- or CD11b-specific monoclonal antibodies and an ALEXA546 (Molecular Probes) secondary goat anti-mouse IgG conjugate. Cell clusters positive for CD3, CD4, CD8 or CD11b were gated using the red fluorescence channel and analyzed for GFP expression in the green fluorescence channel. By histogram analysis, between 13.2% and 22.9% of the analyzed PBMC populations were positive for GFP expression. The percentages of green fluorescing CD4+ and CD8+ T-cells were virtually identical.

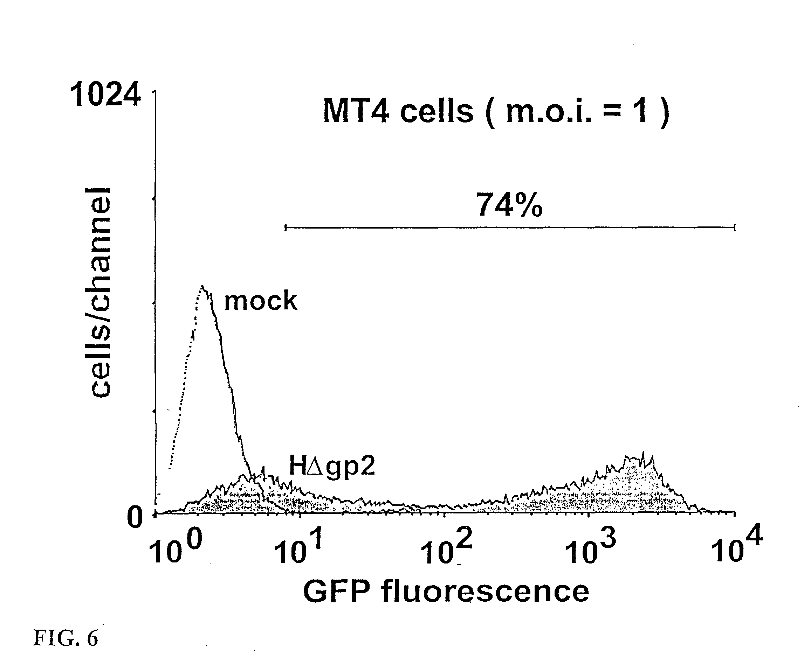

**Figure 6** depicts a flow cytometric histogram analysis of human CD4+ MT4 cells at 24 hr after inoculation (0.5 infectious units per cell) of EHV-1 HΔgp2. Approximately 74% of the HΔgp2 inoculated MT4 cells were positive for the expression of GFP when compared to mock-transduced control cells.

**Figure 7:** GFP expression in murine lungs at day 2 after intranasal application of 1 X 10⁴ IU per mouse. (A) Lungs were removed and immediately scanned *in situ* for GFP expression. Magnification is 100 X. (B) Thin section of a mouse lung after shock-freezing of lungs in liquid N₂. Lungs were left unfixed and unstained to maintain GFP expression. The upper panel shows the light microscopy image of the view shown in the lower panel under the fluorescent microscope. Note the expression of GFP in cells of the bronchioli (arrowhead) and alveolae (arrow). Magnification is 200 X.

The term "heterologous animal" as used herein refers to an animal or cell populations thereof that are infectable by the vector according to the present invention although it is not the natural or a dead-end host for the virus.

The term "vector" as used herein refers to a virus or derivative thereof that is able to transfer a nucleic acid to an animal and humans or cell populations isolated thereof.

The term "replication-deficient EHV" as used herein refers to an EHV based vector that has been genetically modified in a sense that it does not replicate any more in usually permissive cells or cell lines of such as equine or rodent origin e.g. by deletion of a genomic sequence which is essential for viral replication.

The term "replication-competent EHV" as used herein refers to a recombinant virus which is replication competent on permissive cell lines or primary cells.

The present invention relates to the use of animal viruses, which do not use humans as their natural or dead-end host, for therapeutic, preventive or diagnostic gene delivery purposes. Such viruses are found amongst the not limiting group of *Birnaviridae* (e.g. IBDV, Infectious Bursal Disease Virus), *Mononegavirales* (e.g. Newcastle Disease Virus), *Poxviridae* (e.g. Parapox, Capripox, Avipox), *Coronaviridae* (e.g. Transmissible Gastroenteritis Virus, TGEV; Avian Infectious Bronchitis, AIB), *Adenoviridae* (Bovine, Porcine, Canine Adenoviruses) and *Herpesviridae* (Equine Herpesviruses, EHV). The present invention therefore aimed at exploring the potential of such animal viruses, particularly animal herpesviruses and more particularly equine herpesviruses such as EHV-1, for use as a universal vector in heterologous species including human. Surprisingly, the present inventors found that animal herpesvimses exhibit high transduction efficiencies at extremely low particle numbers in a wide variety of heterologous cells, including primary human cells *in vitro* as well as *in vivo.* Noteworthy, this feature combined with those that (i) *EHV like most animal viruses* does not replicate in heterologous organisms being not the natural host, e.g. human cells, that (ii) *EHV* intrinsically does not induce long lasting humoral immune responses, that furthermore (iii) recombinant *EHV* derived vectors can be constructed, which do not express a single *EHV* protein in the transduced or infected cell and that (iv) human sera displaying high antibody titers directed against the most relevant human Herpesviruses do not neutralize *EHV* allows the design of *EHV* derived vectors and gene delivery strategies that allow highly efficient and repeated gene delivery in *vitro* and *in vivo.* At the very same time, such strategies help to avoid the induction of vector immunity and otherwise adverse side effects to the recipient such as toxic, allergic or autoimmune reactions. According to a preferred virus/cell-combination, the herpesvirus is EHV-1 and the cells are *primary* human cells. More preferably, the recombinant EHV-1 is used as a vector to deliver genes to humans *in vivo.*

According to the present invention, a vector system allowing gene delivery to heterologous animals and humans is provided.

A surprising aspect of the present invention relates to the fact that EHV is capable to enter and efficiently transduce a wide variety of cells from different species and primary tissues *in vitro* and *in vivo.* Accordingly, besides infection of various cell lines the inventors could prove infection of primary cells of vertebrate origin including avian e.g. chicken and quail, bovine, porcine, canine, feline and human cells.

Another unexpected aspect of the present invention relates to fact that EHV enters virtually every cell type tested including human cells at extremely high efficiency, and less than one infectious unit equaling approximately 100 particles per cell ^{16,17} are needed to introduce a marker gene such as the enhanced green fluorescent protein (EGFP), into the cell of interest. This aspect is key important to balance optimal gene delivery and at the same time reduce the induction of vector immunity.

Another unexpected finding of the present invention is the fact, that the efficiency of EHV mediated gene delivery of e.g. EGFP into PBMCs can be significantly increased by either specifically stimulating PBMCs by or by unspecific stimulation using mitogens such as e.g. ConA.

Another aspect of the present invention relates to the fact that a marker transgene is expressed efficiently and for prolonged periods in primary stimulated and also unstimulated peripheral blood cells following infection with a recombinant EHV expressing EGFP.

Another surprising aspect of the present invention relates to the fact that marker transgene expression is relatively stably maintained for several continuous passages in cells such as the human CD4+ cell line MT4 without *de novo* production of progeny virus, as was demonstrated by prolonged expression of EGFP in the absence of infectious progeny in supernatants of infected cells. These cells appeared not to be lysed by EHV and obviously resisted productive virus replication, a phenomenon that is most probably caused by episomal and stable maintenance of the EHV genome in some nonpermissive cells.

Another unexpected finding is the absence of neutralizing anti-EHV-1 antibodies in humans, including laboratory personnel who were in constant contact with the agent, and the absence of cross-neutralization by high titer antisera directed against related human viruses such as prominent members of the *Alphaherpesvirinae* group, e.g. HSV-1 and 2, *Betaherpesvirinae* e.g. Human Cytomegalovirus (HCMV), and *Gammaherpesvirinae,* such as e.g. Epstein-Barr virus, both findings representing a necessary prerequisite for efficient use of recombinant EHV as a novel vector in humans for immunizations and gene therapeutic applications. Another finding is that EHV is able to efficiently transduce lung cells in mice *in vivo.* EGFP expression can be detected for more than 12 days after intranasal instillation of low doses of EHV. These properties of EHV make the system e.g. accessible to testing potential therapeutic application in small animal models *in vivo.*

Another surprising aspect is the fact that the *in vivo* administration of EHV based vectors induces only very modest and short lasting humoral responses, which allow repeated application without significant loss in transduction or immunization efficiency.

In sum, EHV based vectors display a variety of advantages over currently used vectors that are derived from viruses that either replicate in humans or at least use humans as their dead end host. Considering these aspects together with the theoretical packaging capacity of EHV, and taking into account the fact that safe vectors expressing foreign genes or containing foreign nucleic acids can be constructed by the introduction of conditionally lethal mutants that can be propagated on complementing/packaging cell lines only¹⁹, the EHV vector system according to the present invention can be exploited in several different ways.

Thus, one object of the present invention relates to a recombinant animal virus, preferably *Birnaviridae* (e.g. IBDV, Infectious Bursal Disease Virus), *Mononegavirales* (e.g. Newcastle Disease Virus), *Poxviridae* (e.g. Parapox, Capripox, Avipox), *Coronaviridae* (e.g. Transmissible Gastroenteritis Virus, TGEV; Avian Infectious Bronchitis, AIB), *Adenoviridae* (Bovine, Porcine, Canine Adenoviruses) and *Herpesviridae* (Equine Herpesviruses, EHV e.g. EHV-1), naturally not using humans as a host, being replication-*competent or* -deficient in and having the ability to transduce primary cells *in vitro* with an MOI of less than 1, said primary cells derived from organisms being not the natural host. Preferably the recombinant animal virus according to the present invention further having the ability to transduce cells *in vivo* at a particle number in the range of approximately 10⁶ to 10⁸ pfu. Preferably the primary cells are derived from human beings. The recombinant animal virus according to the present invention preferably comprises a transgene.

A further aspect of the present invention relates to the use of the recombinant animal virus according to the present invention as a pharmaceutical or diagnostic agent or a vaccine, preferably as a gene targeting vector.

A further aspect of the present invention relates to primary cells transduced with the recombinant animal virus according the present invention.

Such recombinant, replication-competent or -deficient animal viruses according to the present invention can be used to express recombinant proteins in animal, e.g. mammalian, cells of various origin, wherein the organism from which the cells are derived is not the natural host of said virus. Furthermore, such recombinant, replication-competent or -deficient animal virus according to the present invention can be used in immunization strategies as vaccines in animals e.g. domestic or pet animals or humans in order to induce e.g. virus- or tumor-specific immune responses, to generate antigen specific tolerance, anergy or to deplete antigen-specific B-cells, T-cells or distinct subsets thereof for prophylactic or therapeutic purposes. In addition, recombinant, replication-competent or -deficient animal virus, e.g. equine herpesviruses can be used as vectors for somatic gene therapy in humans to restore, compensate or modulate expression of cellular, viral or any other disease related gene(s) to treat genetic disorders such as autosomal recessive inherited genetic diseases, cancer, autoimmune disease or infectious diseases. Such recombinant, replication-competent or -deficient animal virus according to the present invention may be also used as gene delivery systems to generate transgenic animals. Furthermore, the present invention relates to the use of such recombinant, replication-competent or -deficient animal virus according to the present invention expressing selected antigens to diagnose or to follow-up antigen specific T-cell responses that are generated following vaccination, infectious diseases, cancer or autoimmune diseases. Moreover, the present invention allows to compose kits containing at least e.g. a transgene construct containing (i) one of the two known origins of EHV DNA replication, ORI_{S} and ORI_{L,} as well as (ii) the packaging sequences required for incorporation of unit-length viral DNA into preformed capsids, which are provided *in trans* from a packaging cell line in order to generate such recombinant, infectious, however in this case replication-deficient, equine herpesviruses for the above applications. More specifically, such test kits allow the subcloning of gene libraries into the transgene constructs to identify - upon expression in heterologous or syngeneic cells - distinct polypeptides, B and T cell epitopes being relevant for the diagnosis, prevention, therapy and therapy monitoring of infectious and autoimmune diseases, cancer and allergies.

Replication-competent viruses can be constructed as described in materials and methods and illustrated in Fig. 1. Briefly, taking advantage of the BAC cloning and recombination technology, the foreign gene or DNA sequence can be inserted into, or substitute either completely or partially intragenic regions or non-essential genes that can be deleted without affecting or only marginally influencing virus growth. Examples for such nonessential genes are the gp2, gC, gE, or the gI gene (15).

Replication-deficient viruses can be based on the deletion of several open reading frames from the EHV genome, which are essential for viral replication either alone or in combination, such as e.g. the only immediate early gene encoded by EHV-1 gene 64. Especially, because EHV-1 expresses only one immediate early (IE) transactivator, which regulates itself and the expression of all non-structural and structural proteins, deletion of this IE gene represents a very efficient means to construct replication-deficient EHV-1. Using this approach, EHV-1 derived vectors can be constructed ― initially as an EHV-1 based BAC containing the complete EHV-1 genome except the IE gene - that will be transfected into and propagated on a complementing cell line. Such recombinant EHV-1 vectors are able to infect target cells of e.g. human origin. After entering of the target cell *in vitro* or *in vivo,* however, only the transgene under the control of a non-EHV-1-derived, heterologous promoter/enhancer will be expressed and anti-EHV-1 immune responses can completely be avoided.

The gene therapy vector can be further developed by construction of 'gut-less' EHV. Such gut-less EHV preferably EHV-1 derived vectors are essentially based on EHV-1 derived 'replicon' genomes that contain at least one of the two known origins of EHV-1 DNA replication, ORIS and ORIL20-22 and the packaging (pac) sequences required for the incorporation of unit-length viral DNA into preformed nucleocapsids 22-24. These mutant 'replicon' genomes may also contain genes of essential nucleocapsid, tegument or envelope components. Such 'replicon'-genomes can be packaged with the help of cell lines harboring mutant EHV-1 genomes, which lack virus packaging sequences as well as ORIS and/or ORIL, but provide and complement the required and essential genes removed from the vectors for virus DNA packaging in trans.

Because these cell lines do not encode pac sites, only the 'replicon' genomes are packaged and as such represent a pure recombinant virus population

The packaging cell lines can also be constructed in the form of stable cell lines of various origins, where the replication-defective EHV-1 derived 'replicon' genome contains the Epstein Barr Virus (EBV) derived ORI_{P} and therefore segregates into packaging daughter cells that are expressing the EBV derived EBNA-1 protein. EBNA-1 can be stably produced by the complementing cell line after introduction of the gene into the packaging cell using e.g. G-418 resistance for selection. EBNA-1 mediates segregation of the EHV-1 derived 'replicon' genome and its stable maintenance in progeny cells by binding to ORI_{P}²³.

In order to prolong maintenance of the transgene information in the target cells or target tissues, the EBV ORIp and the EBNA-1 ORF can be also inserted into the EHV 'replicon' genome to be packaged in complementing packaging or producer cells into EHV, preferably EHV-1 vector particles. Upon successful transduction of target cells or target tissues with the EHV vector, EHV derived 'replicon' genomes containing the transgene information segregate - mediated by binding of EBNA-1 to ORI_{P} - during mitosis into daughter cells, thereby leading to long term maintainance of EHV derived 'replicon' genome in the nucleus of transduced cells. Alternatively, prolonged maintainence of the transgene information can be also achieved by inserting a matrix attatchment site (MAT) into the EHV derived replicon genome.

Targeting of the EHV derived vector according to the present invention to specific cell types independently from EHV glycoproteins is possible. For example, EHV-1 attachment to cells is mediated by binding of the virion to glycosaminoglycans on the cell surface through gC ¹⁸. Stable attachment and receptor binding is mediated by gD in all *Herpesvirinae* analyzed to date ⁵, while fusion of the viral envelope with the plasma membrane occurs by yet unknown processes involving gB, gD and the gH-gL complex ^{3,24}. However, efficient release of virus from infected cells in the absence of gB, gD and gH-gL has been demonstrated ²⁵.

As an example, pseudotyped EHV vectors can be produced by several means. For example, by deleting gB, gD and gH-gL - either alone or in combination, respectively - from the viral genome and construction of a respective complementing packaging cell line, single or or multiple deleted EHV-1 can be generated. These viruses can be used to infect non-complementing cells from which 'noninfectious' virus is released. In the envelope of this virus progeny, the receptor targeting protein of interest, e.g. gp120/4, mediating either cell type or tissue specificity on a surface exposed membrane receptor level, can be inserted by expression of the respective gene from the cell line. Alternatively, EHV-1 based BAC genomes lacking the gB, gD, gH-gL coding regions can also be directly pseudotyped by e.g. substituting all or part of the said EHV-1 glycoproteins gB, gD and gH-gL by the receptor targeting protein of interest. Such viruses may then be produced easily on a permissive production cell line that expresses the receptor(s) together with potential coreceptor(s). In the example of gp120/41 pseudotyped viruses, receptor and coreceptor molecules are the primary human CD4 receptor together with the human coreceptors e.g. CXCR4 or CCR5. Optionally, in addition to gB, gD and gH-gL, the IE gene and/or other genes can be deleted from the viral genome e.g. to avoid expression of EHV derived gene products in the target cell and/or to increase the packaging size of the EHV derived vector (gutless vectors) and must therefore be complemented in the above described packaging and production cell lines.

Receptor targeting proteins to mediate cell type or tissue specificity can be derived from different sources such as (i) from viruses e.g. the gp120/gp41 envelope protein from HIV-1 supporting the specific transduction of CD4 positive cells by pseudotyped retroviruses, (ii) from cellular receptors known to naturally mediate close cell-to-cell contact, (iii) from soluble ligands known to recognize their cognate cellular receptors such as complement components or chemokines that can be immobilized on the virion surface via a heterologous transmembrane domain and thereby mediate contact to e.g. antigen presenting cells, naive or memory T- or B-cells (depending e.g. on the nature of the selected chemokine), (iv) from genetically engineered, membrane bound antibodies, Fab-fragments or surrogates thereof such as single chain antibodies, derivatives of the PSTI *(pancreatic secretory trypsin inhibitor)* or any alternative scaffold protein that has been selected by e.g. phage or ribosomal display technologies or (v) from parts thereof representing the complementarity determining region(s) or receptor interacting determinants inserted into an appropriate site of a receptor targeting or membrane-fusion mediating protein. Moreover, efficient fusion and entry of e.g. EHV-1 vector particles into target cells may be achieved either independently or assist cell type and tissue specific binding by expression or coexpression of completely or partially non-selective amphotropic, e.g. viral envelope proteins such as the VSV-G envelope protein, MoMLV envelope protein or the GaLV envelope protein.

EHV derived vectors can be used for the delivery of non-EHV vectors such as e.g. retroviral or lentivital vectors. In order to construct such hybrid EHV vectors, the EHV derived 'replicon' genomes to be packaged into EHV derived vector particles have to contain at least the retro- or lentiviral 'replicon' DNA genome encoding the transgene information in addition to the minimal information required for replication of the EHV derived 'replicon' genome and its packaging into EHV vector particles in a complementing packaging cell line. The retro- or lentiviral 'replicon' DNA genome inserted into the EHV 'replicon' genome necessarily has to provide at least all cis-acting elements required for generation of the retro- or lentiviral RNA replicon, its packaging into retro- or lentiviral particles, reverse transcription, nuclear targeting and integration of the lentiviral 'replicon' genome into the host cell genome and expression of the transgene. Such infectious, but non-replicating hybrid EHV based vector particles can be used to produce retro- or lentiviral vector particles by infecting retro- or lentiviral packaging cells that provide the packaging functions such as GagPol and a surface exposed targeting protein, e.g. the VSV G protein *in trans.*

Alternatively, in addition to the retro- or lentiviral 'replicon' DNA genome, the EHV derived 'replicon' genomes to be packaged into EHV derived vector particles can also contain (i) a wild-type or codon optimized retro- or lentiviral gagpol packaging gene as well as (ii) an open reading frame encoding for a surface exposed targeting protein mediating cell specificity of the retro- or lentiviral vector. Such complex hybrid EHV--based vector particles can be also used to produce retro- or lentiviral vector particles *in vitro.* A preferred application of such complex hybrid EHV based vector particles is their use *in situ* or *in vivo,* where ― upon infection of cells with the hybrid EHV based vector particles ― retro- or lentiviral particles are produced *in situ,* which would then allow the stable transduction of target cells by integrating the transgene information into the host cell genome.

Another example for the use of e.g. EHV-1 derived vectors to deliver non-EHV-1 derived vectors is the delivery of an *Alphavirus* (e.g. Semliki-Forest Virus SFV, Sindbis or Venezuela Enzephalitis Virus VEE) replicon to a target cell. Such *Alphavirus* derived replicons usually are regulated by e.g. viral, cell-type-specific or inducible promotors that drive nuclear transcription of a unit that contains sequences encoding alphaviral nonstructural proteins and representing an alphaviral promotor required for strong cytoplasmic transcription of the transgene that is controlled by the transactivating alphaviral nonstructural proteins. An example for the order of these elements on the transcription unit is 5'- CMV-promotor/enhancer― Alphavirusnonstructural proteins ― Alphavirus promotor ― transgene ― 3'. In order to construct such hybrid EHV-1 vectors, the EHV-1 derived 'replicon' genomes to be packaged into EHV-1 derived vector particles should contain at least the above described alphaviral 'replicon' that includes the transgene information in addition to the minimal information required for replication of the EHV-1 derived 'replicon' genome and its packaging into EHV-1 vector particles in a complementing packaging cell line.

Transcription of the transgene information can be driven by any heterologous, e.g. viral, cellular or any sort of hybrid promotor/enhancer unit. Constitutive transcription can be achieved by a variety of viral promotor/enhancer elements such as e.g. the viral Human Cytomegalovirus (HCMV) immediate early promotor/enhancer, the Rous Sarcoma Virus promotor/enhancer or the Adenovirus major/late promotor/enhancer or by cellular promotor/enhancer units driving the expression of housekeeping genes such as the actin promotor. Alternatively, cell type-specific targeting of the transgene expression may be achieved or supported by using cell type or tissue specific promotor/enhancer elements such as those regulating the expression of e.g. the muscle kreatin kinase (MCK), the MHC class I or II molecules, the CD4 receptor, insulin, factors of the blood clotting cascade or others. Alternatively, one can take advantage of inducible promotor/enhancer elements such as e.g. hormone inducible P/E elements or, preferably, promotor enhancer units that can be induced by tetracycline or analogues thereof. The transcription units to be delivered by the EHV-1 based vectors may encode a variety of biologically active compounds such as e.g. polypeptides, apta- or intramers, ribozymes, antisense constructs or small interfering RNAs (siRNAs) for diagnostic, preventive or therapeutic purposes. Such biologically active molecules delivered either alone or in combination by such recombinant EHV-1 derived vectors can be used to induce, to enhance and to modulate immune responses, to break or to induce tolerance, to induce anergy, or to deplete specific T- or B-cells or subsets thereof.

Accordingly, EHV-based vectors may be simply used to produce any sort of polypeptide in cell culture for diagnostic, preventive or therapeutic purposes.

Alternatively, EHV-1 derived vectors may be used to infect or to transduce primary target cells of non-equine animal and human origin or cell lines of non-equine animal and human origin *in vitro and in vivo.*

Any non-equine animal and human cell line or cell population that can be selected, generated, manipulated and cultivated *ex vivo* may represent a target cell to be transduced *in vitro.* Preferably, these cells are primary cells such as primary immune cells isolated from animals e.g. livestock or pets or humans, more preferably pluripotent progenitor cells, embryonic stem cells, bone marrow stem cells, blood derived stem cells, hematopoietic stem cells, B cells, T-cells or distinct subsets or derivatives thereof. Most preferably, these cells are antigen-presenting cells such as B-cells, cells of the monocyte/macrophage lineage, dendritic cells or derivatives thereof, respectively. Alternatively, these cells are primary tumor cells.

Successfully transduced cells can be selected taking advantage of positive or negative selectible markers such as dihydrofolate reductase (DHFR) or neomycin, or using immunological markers such as NGFR, for e.g. fluorescence activated cell sorting (FACS) or physical enrichment of transduced cells using the magnetic bead technology as being commercially available by Milenyi BioTech or Dynal. Selected cells may be either expanded themselves or, more specifically, can be used to expand immune cells such as antigen specific T-cells. Transduced cells can be further propagated in culture, e.g. to purify secreted components such as polypeptides from the cell culture supernatants. Preferably, transduced cells can be reintroduced into humans, pets, livestock or laboratory animals for therapeutic or preventive purposes.

Preferably, *ex vivo* transduction of cells, preferably of primary and most preferably of primary human cells using recombinant, *replication-competent or* -deficient animal virus according to the present invention can be used to deliver selected, disease relevant antigens, chimeric or hybrid proteins, epitopes or epitope strings representing e.g. viral antigens, tumor antigens, antigens or epitopes accounting for autoimmunity or allergic reactions into cells, more specifically to antigen presenting cells and preferably to dendritic cells or derivatives thereof, respectively, in order to achieve favourable presentation of relevant epitopes such as e.g. MHC class-I and class-II restricted T-cell epitopes and/or B cell epitopes to the immune system. The outcome of the strength and type of immune response can be influenced and modulated e.g. by co-expressing in the transduced cells such as e.g. dendritic cells selected cytokines such as e.g. IL-2, gIFN, IL-12, IL18, IL-10, IL-4, IL-6 (either alone or in combinations) and/or chemokines such as e.g. Rantes, MIP1a, MIP1ß, SDF-1, SLC, ECL, BCA-1, DC-CK1, IP-10 or derivatives thereof (either alone or in combinations) to influence the Th1/Th2 balance, to attract defined subsets of immune cells and to break tolerance. Alternatively or supplementary, the expression levels of cellular surface receptors or ligands involved in intracellular signaling such as costimulatory signals e.g. B7.1, B7.2, CD80, CD86 can be modulated in transduced cells e.g. by co-transcribing reading frame specific ribozymes, antisense RNAs or siRNAs to switch from the induction of immunity to e.g. peripheral T-cell tolerance by inducing anergy. Furthermore, by employing the fas/fasL system in transduced cells, antigen specific T-cells may be depleted thus phenotypically also leading to tolerance.

Alternatively, *ex vivo* transduction of cells using recombinant, replication-competent or deficient animal virus according to the present invention can be used as vectors for somatic gene therapy in humans to restore, compensate or modulate expression of cellular, viral or any other disease related gene(s), to treat genetic disorders such as autosomal recessive inherited genetic diseases, cancer, autoimmune disease or infectious diseases by delivering e.g. transdominant negative mutants, molecular decoys, ribozymes, antisense constructs or siRNAs. More specifically, transduced embryonic stem cells may be used to generate transgenic animals such as e.g. mice and live stock.

Transduction of primary human cells *in vitro* can be also used for diagnostic purposes, e.g. to identify, to specify and to monitor humoral, and ― preferably ― cellular, e.g. MHC class I- and MHC class II-restricted and, in particular, CD4- and/or CD8-positive T-cell responses. For example, purified PBMCs, preferably purified antigen-presenting cells and most preferably purified dendritic cells or derivatives thereof may be infected by recombinant replicating or replication-incompetent EHV-1 expressing the immunological target protein or derivatives thereof. Alternatively, whole blood samples may be directly subjected towards infection of susceptible cells with recombinant EHV-1 expressing the transgene of interest to achieve MHC class II- and/or MHC class I-restricted presentation of selected epitopes to immune cells. Subsequent stimulation of antigen specific T-cells may be monitored by different means such as e.g. determination of T-cell proliferation via incorporation of e.g. ³H thymidine or bromodesoxyuridine, quantification of cytokine and chemokine producing cells in an ELISPOT assay or, alternatively, by FACS analysis, or by setting up a standard chromium release assay. Furthermore, gene libraries obtained e.g. as a result from subtracting cDNAs of different cell populations from each other may be cloned into replication-competent or non-replicating EHV-1 I derived vectors to generate a vector population that is then further subjected to detailed immunological analysis according to above outlined procedure to identify and to characterize MHC class I- and MHC class II-restricted T-cells being involved in autoimmune disease, tumor rejection and control of infectious diseases.

Taking advantage of the lack of EHV specific preexisting immunity in humans, pets and livestock and considering the efficient infection of e.g. primary human cells at low particle numbers, another preferred embodiment of the present invention is the direct administration of EHV- derived vectors to humans or pets or live-stock *in vivo* and *in situ* without prior *ex vivo* transduction of target cells for preventive or therapeutic purposes.

In particular, our findings demonstrating that extremely low multiplicities of infection allow efficient infection of primary human cells such as B and T cells and that MOIs <1 are sufficient to effectively infect human cell lines *in vitro,* strongly suggest that *in vivo* gene delivery by EHV vector particles is approximately 100- to 5000-fold more efficient than adenovirus mediated gene delivery. Accordingly, a major advantage of using such highly effective, EHV-based vectors at low particle numbers (10⁶-10⁸ pfu; 100- to 5000-fold lower as compared to adenoviral vectors) for *in vivo* gene delivery to man, pets and livestock is its ― compared e.g. to adenoviral vectors-low potential to induce toxic side effects, allergies or autoimmune diseases.

Such comparably low numbers of EHV-derived, non-replicating vector particles contribute to limit immune responses directed against the viral vector. This fact together with the *per se* short-lived EHV specific humoral immune responses allow repeated booster immunizations at short intervalls using the very same EHV strain as vector and thus enable the usage of EHV derived vectors as universal vehicles for the delivery of various antigens, genes or DNA sequences.

In the case of non-replicating animal virus derived vectors according to the present invention, this effect can be further extended, when e.g. the packaged EHV replicons lack e.g. the EHVencoded immediate early gene 64. Such IE-gene deprived, EHV derived vectors allow infection and transduction of target cells *in vivo* without expressing any EHV protein thus avoiding the elimination of successfully infected or transduced cells by EHV specific immune responses.

Besides that, EHV-derived vectors display a tendency to preferably transduce non-polarized cells such as PBMCs and tumor cells.

Accordingly, in addition to the transduction of target cells *ex vivo,* such recombinant, replication-competent or -deficient equine herpesviruses can be also used directly *in vivo* or *in situ* in immunization strategies as vaccines in animals, e.g. pets and livestock, and humans in order to induce e.g. virus- or tumor-specific immune responses, to generate or break antigen-specific tolerance, to induce anergy or to deplete antigen-specific B-cells, T-cells or distinct subsets thereof for prophylactic or therapeutic purposes.

Direct *in situ* or *in vivo* administration of such recombinant, replication-competent or -deficient animal virus according to the present inventioncan be used to deliver selected, disease relevant antigens, chimeric or hybrid proteins, epitopes or epitope strings representing e.g. viral antigens, tumor antigens, antigens or epitopes accounting for autoimmunity or allergic reactions to the immune system, more specifically to antigen presenting cells and most preferably to dendritic cells or derivatives thereof, respectively, in order to achieve favorable presentation of relevant epitopes such as e.g. MHC class-I and class-II restricted T-cell epitopes and/or B cell epitopes to the immune system. The outcome of the strength and type of immune response can be influenced and modulated e.g. by co-expressing selected cytokines such as e.g. IL-2, gIFN, IL-12, IL-18, IL-10, IL-4, IL-6 (either alone or in combinations) and/or chemokines such as e.g. Rantes, MIP1a, MIP1ß, SDF-1, SLC, ECL, BCA-1, DC-CK1, IP-10 or derivatives thereof (either alone or in combinations) to influence the Th1/Th2 balance, to attract defined subsets of immune cells and to break tolerance. Alternatively or supplementary, the expression levels of cellular surface receptors or ligands involved in intracellular signaling such as costimulatory signals e.g. B7.1, B7.2, CD80, CD86 can be modulated in transduced cells e.g. by co-transcribing reading frame specific ribozymes, antisense RNAs or siRNAs to switch from the induction of immunity to e.g. peripheral T-cell tolerance by inducing anergy. Furthermore, by employing the fas/fasL system in transduced cells, antigen specific T-cells may be depleted thus phenotypically also leading to tolerance.

In addition, recombinant, replication-competent or -deficient animal virus according to the present invention can be also used *in vivo* as vectors for somatic gene therapy in humans to restore, compensate or modulate expression of cellular, viral or any other disease related gene(s) to treat genetic disorders such as autosomal recessive inherited genetic diseases, cancer, autoimmune disease or infectious diseases.

Different routes of administration can be used to deliver the desired antigens, transdominant negative gene mutants, ribozymes, antisense RNAs or siRNAs to the recipient. The spectrum of delivery routes ranges from (i) *in situ* administration e.g. by injecting recombinant, replication-competent or -deficient equine herpesviruses into the vena porta in order to transduce liver cells, (ii) inhalation to deliver genes into the lung, (iii) intranasal, rectal or vaginal administration e.g. to generate mucosal responses, (iv) subcutaneous or intracutaneous injection to target specific antigen presenting cells, (v) intramuscular administration until (vi) intravenous injection.

Another way to deliver antigens or nucleic acids to the immune system is to (i) either encapsulate cells following infection with a recombinant, replication-competent or -deficient equine herpesvirus or (ii) to infect encapsulated or otherwise immobilized or compartmented cells with recombinant, *replication-competent or* -deficient animal virus according to the present invention and to administer such infected, encapsulated or immobilized cells then to the recipient either systemically or *in situ* by addressing specific organs. Such cells may either be (i) packaging cells supporting the production of infectious, however, replication-deficient animal virus according to the present inventionor (ii) syngenic cells, cell populations or cell lines allowing appropriate expression and secretion of the desired antigens.

Accordingly, it was one of the objects of the present invention to analyze the capacity of EHV-1 to serve as a universal vector for the delivery of DNA sequences encoding e.g. a transgene reporter into different cells of non-human and human origin in vitro. More specifically, an object of the present invention is to analyze the capacity of an EHV-1 to serve as a universal vector for the expression of genes in vitro and in vivo to immunize against infectious diseases of man and the most important mammal and avian species. Another objective of the present invention is to analyze the capacity of EHV-1 to be used as a human anti-tumor vaccine. Moreover, it was an objective of the present invention to analyze the capacity of EHV-1 derived vectors to be used for stable transduction of cells with large pieces of DNA for either gene therapy purposes, generation of transgenic animals or diagnostic purposes. Another object of the present invention is to analyze the capacity of EHV-1 to serve as a universal vector for the in vitro application and the functional testing of BAC/PAC libraries of the murine, human or any other genome in various cell types^{*23*}

Especially with regard to the high packaging capacity, the extremely efficient delivery of a marker gene at low virus numbers per cell, the lack of neutralization by human sera, its ability to establish latent infection in actively replicating cells, and its capacity to mediate efficient and sustained transgene expression *in vivo* in a murine model, EHV-1 may prove an extremely useful novel tool for use as a vector in animals and humans for diagnostic, preventive and therapeutic purposes.

There is a number of advantages of EHV-1 over other systems to be used as vectors in man, pets and live-stocks, that - if exploited and applied systematically ― generate a novel delivery system with hitherto unknown properties. These include (1) its capability to very efficiently infect heterologous species such as man at extremely low particle numbers, (2) the absence of neutralizing anti-EHV-1 antibodies and (3) the absence of cross-neutralization of related human viruses, the fact that (4) EHV per se induces only minor vector specific immune responses, which (5) can be further decreased by generating safe, conditionally lethal mutants that can be propagated on complementing/packaging cell lines¹⁹ and that express no single EHV derived protein in the recipient cell, (6) the theoretical packaging capacity of EHV-1 which by far exceeds 100 kbp, as well as (7) the fact that EHV-1 expressing or delivering foreign nucleic acids can easily be tested in small animal (rodent) models²⁴.

In order to accomplish the present invention, the inventors made use of the following materials and methods.

### Materials

**Viruses and cells.** Derivatives of the avirulent EHV-1 vaccine strain RacH (256^{th} passage,²⁶) were used and grown on RK13 cells which were propagated in Dulbecco's minimal essential medium (DMEM) supplemented with 10% fetal calf serum (FCS). In all infection experiments, avirulent RacH virus reconstituted from infectious BAC clone pRacH was used (Fig. 1). The virus reconstituted from pRacH lacks the glycoprotein gp2 gene (gene 71) and was termed HΔgp2^{7,8}. As a control virus, GFP-expressing Bovine Herpesvirus Type 1 (BHV-1), strain Schönböken, was used. In case of BHV-1, virus was reconstituted from infectious BAC clone pBHV-1. BHV-1 reconstituted from pBHV-1 was termed BHV-1ΔgE and lacks the gE open reading frame (ORF), instead of which the mini F plasmid pHA2 including the GFP expression cassette was inserted.

Human cell lines were the CD4+ T cell line MT4 (MRC ARP017), the human melanoma cell line MeWo (ATCC HTB-65), the hepatocellular carcinoma cell line HuH7 (ATCC CCL-185), kidney carcinoma cell line 293 (ATCC CRL-1573), lung carcinoma cell line H1299, and human cervix carcinoma cell line HeLa (ATCC CCL-2). We also used bovine Madin Darby Bovine Kidney (MDBK; ATCC CCL-22) cells, Madin Darby Canine Kidney (MDCK; ATCC CCL-34), feline embryo cells (collection of cell lines in veterinary medicine at the Federal Research Center of Virus diseases of Animals Insel Riems; RIE138), porcine kidney cell line PK15 (ATCC CCL-33) as well as primary chicken embryo cells (CEC) and quail muscle QM7²⁷. The cell lines were maintained in DMEM supplemented with 10% FCS. Primary human, porcine, bovine or equine peripheral blood mononuclear cells (PBMC) were prepared as follows. Ten milliliters of venous blood or blood from the umbilical cord were sampled into 2 ml of a 2% sodium citrate solution. PBMC or umbilical stem cells were isolated after density gradient centrifugation using Ficoll (Histopaque®, Sigma). PBMC were washed twice in phosphate-buffered saline (PBS) and suspended in RPMI (Gibco-BRL). PBMC were either left untreated or stimulated with concanavalin A (ConA) at a concentration of 5 µg per ml for 24 to 48 hr before inoculation with HΔgp2 or BHV-1ΔgE virus.

### Methods

**Single-step growth kinetics and virus titer determinations.** Single step growth kinetics were determined after infection of 1 X 10⁵ cells seeded in 24-well plates at a multiplicity of infection (m.o.i.) of 3. Virus was allowed to attach for 2 hr on ice, followed by a penetration period of 1 hr at 37°C. At the indicated times after the temperature shift, supernatants of infected were harvested, and viral titers in both preparations were determined by plating onto RK13 cells (EHV-1) or MDBK cells (BHV-1).

**Indirect immunofluorescence and flow cytometry.** For indirect immunofluorescence (IF), cells were grown in 24- or 6-well plates and subsequently infected with GFP-expressing HΔgp2 at various m.o.i.'s. Expression of GFP and binding of antibodies detected with secondary antibodies conjugated with various fluorochromes was visualized using inverted fluorescent microscopes (Zeiss Axiovert and Olympus) or by flow cytometry using the FACScan (Becton-Dickinson). Virus-infected cells were transferred to a U-bottom 96 well microtiter plate (4 x 10⁴cells/well). Monoclonal antibodies (mabs) directed against various human CD markers (anti-CD3, anti-CD4, anti-CD8, anti-CD11b, anti-CD16, anti-CD319; all antibodies from Dako) were added for 30 min on ice, and cells were washed twice with PBS. ALEXA543 goat anti-mouse IgG conjugate (Molecular Probes; USA) was added for 15 min. After two additional washes with PBS, cells were resuspended in 100 µl PBS and analyzed by flow cytometry (at least 5000 cells/sample)²⁸.

### Virus neutralization assays

Virus suspensions containing 10 to 100 pfu were incubated in 10% DMEM with human antisera containing high titers of VZV-, HSV-1-, HCMV- or EBV-specific antibodies for 1 to 4 hr at 37°C. Controls included equine or murine sera which were negative or positive for EHV-1 antibodies. Triplicates of the virus-antibody mixture was added to RK13 cells and incubated for 48 to 96 hr at 37°C before cytopathic effects were analyzed by light microscopy.

**Animal experiments.** BALB/c or C57/black were inoculated intranasally with 2X10⁴ infectious units of EHV-1 (in 20 µl) after ether anesthesia¹⁸ . At 1 to 14 days after instillation, mice were killed and lungs were removed and immediately examined *in situ* for expression of GFP. Lungs were then shock-frozen in liquid nitrogen and thin sections were prepared. No fixation was done to avoid depression of the GFP fluorescent signal. Lung sections were scanned by light and fluorescent microscopy. Pictures were taken with a digital camera (Olympus).

### Example 1: EHV-1 efficiently infects cell lines of various origin and delivers foreign genes into cells

It has previously been shown that EHV-1 can efficiently infect and replicate in cells of equine and rodent origin. Substrates for growth of EHV-1 are primary equine cells derived from nasal epithelium, lung, thyroid gland and skin^{18,26}. In addition, EHV-1 strains have been shown to grow to high titers in rabbit RK13 cells, mouse LM cells as well as baby hamster kidney BHK-21/C13 cells^{1,26,29,30}. It could also be demonstrated that foreign genes are efficiently expressed under a variety of promoters in these cells^{17,26,29,31}.
We were interested to explore the ability of gp2-negative EHV-1 HΔgp2 expressing GFP under control of the HCMV immediate early promoter/enhancer to infect cells of avian, bovine, porcine, canine, feline and primate origin. We were able to demonstrate that EHV-1 entered cells of avian, bovine, porcine, canine, feline and human origin efficiently. As analyzed by fluorescent microscopy, in case of CEC, QM7, MDBK, PK15, MDCK, CRFK and the human cell lines MT4, HeLa, HuH7, 293, H1299 and MeWo, between 2 and 100% of the cells were shown to express GFP from 24 h p.i. (Fig. 2). In contrast, BHV-1ΔgE recovered from infectious plasmid pBHV-1 was unable to infect cells of non-bovine origin efficiently, indicating that EHV-1 has a unique ability among *Alphaherpesvirinae* of non-human origin to infect human cell lines.

### Example 2: EHV-1 efficiently infects porcine and bovine PBMC

In the next series of experiments we investigated the capacity of EHV-1 to enter primary cells obtained from peripheral blood of important livestock species. Venous blood was taken from horses, pigs, or bovines, and infected at an m.o.i. of 0.1. Whereas approximately 0.5% of PBMC of theses species were expressing GFP upon inoculation of EHV-1, 5 to 25 % of the PBMC were shown to express GFP when cells were infected 24 to 48 hr after stimulation with ConA (Fig. 3). It is interesting to note that equine PBMC which have been described to be both a site of lytic infection and latency of EHV-1 in the horse exhibited a similar susceptibility to infection with HΔgp2 as was determined for PBMC isolated from the other species. The results demonstrated that EHV-1 is able to efficiently enter PBMC of livestock animals. In contrast, BHV-1ΔgE was not able to enter PBMC of either species as reflected by the absence of GFP-expressing cells after addition of virus to stimulated or unstimulated PBMC.

### Example 3: EHV-1 efficiently transduces human PBMC and umbilical stem cells

The above results on the ability of PBMC of other species to be transduced by EHV-1 were very surprising. Therefore, PBMC of different female and male human individuals, as well as umbilical cord stem cells from a male and a female newborn, respectively, were obtained and cultured. PBMC or stem cells were maintained in RPMI supplemented with 10% FCS or in medium containing ConA. It could be shown that PBMC of a total of 4 individuals could be infected with EHV-1 and that expression of GFP occurred. In addition, stem cells from a male and female fetus could be efficiently transduced using EHV-1 and more than 15% of isolated stem cells expressed GFP. As reported for equine, porcine, and bovine PBMC, infection and GFP expression was more effective if PBMC were stimulated for 24 h using ConA (Fig. 4). As determined by flow cytometry, approximately 1% of PBMC were transduced with EHV-1 in non-stimulated cultures, and up to 22% of PBMC were GFP positive at 24 h after infection of ConA stimulated blasts. As described above, BHV-1ΔgE was not able to enter either stimulated or non-stimulated human PBMC. These results and those described above demonstrated that EHV-1 can efficiently transduce cell lines of various origins and that it is able to enter human PBMC at high efficiency.

### Example 4: EHV-1 mediates gene transfer into human CD3+, CD4+, CD8+ and CD11b+, and CD19+ cells with similar efficiency

We next sought to identify the nature of human PBMC transduced by EHV-1. EHV-1 was used to infect human PBMC stimulated for 24 h with ConA or left non-stimulated at an m.o.i. of 1. Cells were incubated at 24 h p.i. with specific CD markers and flow cytometry was performed. It could be demonstrated that human B lymphocytes (CD19+), and both CD4+ and CD8+ human T lymphocytes were efficiently transduced using EHV-1 as demonstrated by the appearance of double-labeled cells after detection of the respective differentiation molecules on the surface of GFP-expressing cells at 24 h p.i., and approximately 15 to 22 % of cells of each of the tested phenotypes were shown to express the marker GFP gene (Fig. 5). In addition, expression of GFP was readily detected in approximately 10% of CD11b+ cells (monocytes, granulocytes and natural killer cells) grown out from the suspension culture (Fig. 5). Furthermore, CD4+ MT4 cells also could be transduced at a high efficiency (Fig. 6). The results demonstrated that EHV-1 is able to transduce a wide variety of human PBMC after stimulation with ConA and at low m.o.i. equaling less than 100 particles per cell ^{1,18}.

### Example 5: EHV-1 is not neutralized by human sera containing high titers of neutralizing antibodies against Human Herpesviruses

In order for EHV-1 to be used in human vaccination or for gene therapy, we addressed the question of neutralization of EHV-1 by sera containing high titers of antibodies directed against the *Alphaherpesvirinae* HSV-1 or VZV, as well as the *Betaherpesvirus* HCMV, and the *Gammaherpesvirus* EBV. In addition, sera of three investigators routinely working with EHV-1 were tested. Sera were inactivated for 30 min at 56°C or were left untreated, and 100 or 10 p.f.u. of EHV-1 strain RacH or the HΔgp2 mutant virus in 100 µl were incubated with log2 dilutions (100 µl) of the human sera in DMEM for 1 to 4 hr at 37°C. The virus-antisera mixtures were then mixed with RK13 cells. In none of the human sera could any neutralizing activity against EHV-1 be detected, and complete cytopathic effect developed even if the lowest sera dilutions (1:4) and only 10 p.f.u. of virus were used (Table 1). In contrast, murine or equine sera immunized once with RacH³² readily neutralized 10 or 100 p.f.u. of both RacH and HΔgp2 exhibiting titers of 1:16 to 1:256 (Table 1). These results demonstrated that EHV-1 cannot be neutralized by human sera, even if they contain high titers against Human Herpesviruses. In addition, constant exposure of humans of both genders to EHV-1 under laboratory conditions did not result in induction of EHV-1-specific antibody titers.

### Example 6: Efficient and sustained transgene expression in vivo by EHV-1.

For application of the newly developed vector in vaccinations or gene therapy applications, expression of the inserted transgene(s) mediated by the vector in *vivo* is imperative. Therefore, expression of GFP *in vivo* after intranasal instillation of 2 X 10⁴ IU of EHV-1 into mice of various genetic backgrounds was assessed. From day 1 to 12 after intranasal application, GFP expression in lungs of both BALB/c and C57/black mice was readily observed. Multiple regions that exhibited high levels of autofluorescence were identified when lungs were screened under the microscope immediately after removal from the bodies (Fig. 5a). In thin sections, high level GFP expression was observed in numerous cells of bronchioli and alveolae. Long-lasting expression of the transgene in airway epithelia, which was detected until day 12 after application of the vector, confirmed the above documented observations on native organs (Fig. 5b). It is important to stress that none of the mice having received the GFP-expressing EHV-1 vector showed any sign of illness during the observation period, because an avirulent and genetically attenuated virus was used. These results indicated that EHV-1 is able to efficiently transduce cells of non-equine origin *in vivo* without causing any clinical symptoms. Taken together the above examples clearly demonstrate that primary peripheral blood mononuclear cells (PBMCs) isolated from cattle, pigs and humans can efficiently be infected with EHV-1. This property of EHV-1 was shown by using a recombinant virus expressing the enhanced green fluorescent protein (EGFP) under the transcriptional control of the HCMV immediate early promoter/enhancer. Human PBMCs could be infected at an efficiency of up to 25 % of isolated, Ficoll gradient-purified and concanavalin A-stimulated cells, whereas approximately 0.5% to 2% of unstimulated cells were infectable. Cells with various phenotypic markers (CD3+, CD4+, CD8+, CD11b+, and CD19+) could be infected at almost identical efficiency. In addition, using multiplicities of infections of <1, virtually 100% of CD4+ human cell line MT4, human melanoma cell line MEWO, human hepatocellular carcinoma cell line HuH7, human kidney carcinoma cells 293, and human cervix carcinoma cell line HeLa were infectable with EHV-1. When infected cells were propagated after infection, GFP expression could be detected for several continuous passages. However, cells did not die following virus infection and no release of infectivity into the cell culture supernatant was observed. These results indicate establishment of a nonlytic EHV-1 infection in non-permissive cells. In addition, EHV-1 was able to enter porcine kidney PK15, bovine Madine Darby bovine kidney cells (MDBK), feline embryo cells (KE-R), chicken embryo cells, and quail muscle cells QM7 with high efficiency. Because herpesviruses including EHV-1 exhibit a high packaging capacity (>100 kbp) and because EHV-1 can establish latent infections in replicating cells, the use of EHV-1 as a universal vector for prophylactic and therapeutic delivery of foreign nucleic acid sequences with regard to immunizations, anti-tumor therapies, treatment of autoimmune diseases and gene therapy in animals and man is possible.

### References

1. O'Callaghan, D.J. & Osterrieder, N. in Encyclopedia of Virology, 2^{nd} edition. eds. Webster, R.G. & Granoff, A. (Academic Press, Harcourt Brace & Co., San Diego, 1999).
2. Telford, E.A., Watson, M.S., McBride, K. & Davison, A.J. The DNA sequence of equine herpesvirus-1. *Virology* **189**, 304-316 (1992).
3. Roizman, B. & Sears, A.E. in Virology, 3^{rd} ed. Fields, B.N., Knipe, D.M., & Howley, P.M. 2231-2295 (Lippincott-Raven Press, Philadelphia, New York; 1996).
4. Davison, A.J. & Scott, J.E. The complete DNA sequence of varicella-zoster virus. *J*. *Gen*. *Virol.* **67**, 1759-1816 (1986).
5. Montgomery, R.I., Warner, M.S., Lum, B.J. & Spear, P.G. Herpes simplex virus-1 entry into cells mediated by a novel member of the TNF/NGF receptor family. *Cell* **87**, 427-436 (1996).
6. Whitbeck, J.C., Peng, C., Lou, H., Xu, R., Willis, S.H., Ponce de Leon, M., Peng, T., Nicola, A.V., Montgomery, R.I., Warner, M.S., Soulika, A.M., Spruce, L.A., Moore, W.T., Lambris, J.D., Spear, P.G., Cohen, G.H., & Eisenberg, R.J. Glycoprotein D of herpes simplex virus (HSV) binds directly to HVEM, a member of the tumor necrosis factor receptor superfamily and a mediator of HSV entry. *J. Virol.* **71**, 6083-6093 (1997).
7. Rudolph, J., O'Callaghan, D.J. & Osterrieder,N. Cloning of the genomes of equine herpesvirus type 1 (EHV-1) strains KyA and racL1 as bacterial artificial chromosomes (BAC). *J. Vet. Med. B Infect. Dis. Vet. Public Health* **49**, 31-36 (2002).
8. Rudolph,J. & Osterrieder,N. Equine Herpesvirus Type 1 Devoid of gM and gp2 Is Severely Impaired in Virus Egress but Not Direct Cell-to-Cell Spread. *Virology* **293**, 356-367 (2002).
9. Brune, W., Messerle, M., & Koszinowski, U.H. Forward with BACs: new tools for herpesvirus genomics. *Trends Genet.* **16**, 254-259 (2000).
10. Borst, E.M., Hahn, G., Koszinowski, U.H., & Messerle, M. Cloning of the human cytomegalovirus (HCMV) genome as an infectious bacterial artificial chromosome in Escherichia coli: a new approach for construction of HCMV mutants. *J. Virol.* **73**, 8320-8329 (1999).
11. Flotte, T.R. Size does matter: overcoming the adeno-associated virus packaging limit. *Respir. Res.* **1**, 16-18 (2000).
12. Bruning, A., Kohler, T., Quist, S., Wang-Gohrke, S., Moebus, V.J., Kreienberg, R., & Runnebaum, I.B. Adenoviral transduction efficiency of ovarian cancer cells can be limited by loss of integrin beta3 subunit expression and increased by reconstitution of integrin alphavbeta3. *Hum. Gene Ther.* **12**, 391-399 (2001).
13. Kianmanesh, A., Hackett, N.R., Lee, J.M., Kikuchi, T., Korst, R.J., & Crystal, R.G. Intratumoral administration of low doses of an adenovirus vector encoding tumor necrosis factor alpha together with naive dendritic cells elicits significant suppression of tumor growth without toxicity. *Hum. Gene Ther.* **12**, 2035-2049 (2001).
14. Molinier-Frenkel, V., Le Boulaire, C., Le Gal, F.A., Gahery-Segard, H., Tursz, T., Guillet, J.G., & Farace, F. Longitudinal follow-up of cellular and humoral immunity induced by recombinant adenovims-mediated gene therapy in cancer patients. *Hum. Gene Ther.* **11**, 1911-1920 (2000).
15. Molinier-Frenkel, V., Gahery-Segard, H., Mehtali, M., Le Boulaire, C., Ribault, S., Boulanger, P., Tursz, T., Guillet, J.G., & Farace, F. Immune response to recombinant adenovirus in humans: capsid components from viral input are targets for vector-specific cytotoxic T lymphocytes. *J*. *Virol.* **74**, 7678-7682 (2000).
16. Neubauer, A., Beer, M., Brandmuller, C., Kaaden, O.R., & Osterrieder, N. Equine herpesvirus 1 mutants devoid of glycoprotein B or M are apathogenic for mice but induce protection against challenge infection. *Virology* **239**, 36-45 (1997).
17. Osterrieder, N., Kaaden, O.R. & Neubauer, A. Structure and function of equine herpesvirus glycoproteins - a review. R&W Publications. Proceedings of the 8th International Conference on Equine Infectious Diseases, 111-118. 1999. Newmarket, UK.
18. Osterrieder, N. Construction and characterization of an equine herpesvirus 1 glycoprotein C negative mutant. *Virus Res*. **59**, 165-177 (1999).
19. Buczynski, K.A., Kim, S.K. & O'Callaghan,D.J. Characterization of the transactivation domain of the equine herpesvirus type 1 immediate-early protein. *Virus Res.* **65**, 131-140 (1999).
20. Yalamanchili, R.R. & O'Callaghan, D.J. Organization and function of the ORIs sequence in the genome of EHV-1 DI particles. *Virology* **179**, 867-870 (1990).
21. Yalamanchili, R.R., Raengsakulrach, B., Baumann, R.P. & O'Callaghan,D.J. Identification of the site of recombination in the generation of the genome of DI particles of equine herpesvirus type 1. *Virology* **175**, 448-455 (1990).
22. Yalamanchili, R.R. & O'Callaghan, D.J. Sequence and organization of the genomic termini of equine herpesvirus type 1. *Virus Res.* **15**, 149-161 (1990).
23. Wade-Martins, R., Frampton, J., & James, M.R. Long-term stability of large insert genomic DNA episomal shuttle vectors in human cells. *Nucleic Acids Res*. **27**, 1674-1682 (1999).
24. Neubauer, A., Braun, B., Brandmuller, C., Kaaden, O.R. & Osterrieder, N. Analysis of the contributions of the equine herpesvirus 1 glycoprotein gB homolog to virus entry and direct cell-to-cell spread. *Virology* **227**, 281-294 (1997).
25. Granzow, H., Klupp, B.G., Fuchs, W., Veits, J., Osterrieder, N., & Mettenleiter, T.C. Egress of alphaherpesviruses: comparative ultrastructural study. *J*. *Virol.* **75**, 3675-3684 (2001).
26. Hübert, P.H., Birkenmaier, S., Rziha, H.J. & Osterrieder, N. Alterations in the equine herpesvirus type-1 (EHV-1) strain RacH during attenuation. *Zentralbl. Veterinarmed. [B]* **43**, 1-14 (1996).
27. Schumacher, D., Tischer, B.K., Fuchs,W. & Osterrieder, N. Reconstitution of Marek's disease virus serotype 1 (MDV-1) from DNA cloned as a bacterial artificial chromosome and characterization of a glycoprotein B-negative MDV-1 mutant. *J. Virol.* ***74****,* 11088-11098 (2000).
28. Beer, M., Wolf, G., Pichler, J., Wolfmeyer, A. & Kaaden, O.R. Cytotoxic T-lymphocyte responses in cattle infected with bovine viral diarrhea virus. *Vet. Microbiol.* **58**, 9-22 (1997).
29. O'Callaghan, D.J., Cheevers, W.P., Gentry, G.A. & Randall, C.C. Kinetics of cellular and viral DNA synthesis in equine abortion (herpes) virus infection of L-M cells. *Virology* **36**, 104-114 (1968).
30. Sun, Y., MacLean, A.R., Dargan, D. & Brown, S.M. Identification and characterization of the protein product of gene 71 in equine herpesvirus 1. *J. Gen. Virol.* **75** ( **Pt 11**), 3117-3126(1994).
31. Sun, Y. & Brown, S.M. The open reading frames 1, 2, 71, and 75 are nonessential for the replication of equine herpesvirus type 1 in vitro. *Virology* **199**, 448-452 (1994).
32. Osterrieder, N., Seyboldt, C., & Elbers, K. Deletion of gene 52 encoding glycoprotein M of equine herpesvirus type 1 strain RacH results in increased immunogenicity. *Vet. Microbiol.* **81**, 219-226 (2001).

## Claims

1. Recombinant animal virus derived from a virus which naturally not uses humans or other animal species as a host or dead-end host, being replication-competent or -deficient in and having the ability to transduce primary cells *in vitro* with a multiplicity of infection of less than 1, said primary cells derived from organisms being not the natural or dead-end host.

2. The recombinant animal virus according to claim 1, further having the ability to efficiently transduce cells in *vivo* at low particle numbers in the range of less than 10⁶ to 10⁸ particles per organism

3. The recombinant animal virus according to claim 2, wherein the transduction results in a biologically measurable induction of an immune response, expression of a transgene product sufficient to induce preventive or therapeutic or diagnostic effects in the treated organism.

4. The recombinant animal virus according to any of the preceding claims, said virus being an equine herpesvirus.

5. The recombinant animal virus according to any of the preceding claims, wherein said primary cells are derived from human beings, pet animals or livestock.

6. The recombinant animal virus according to any of the preceding claims comprising a transgene.

7. The recombinant animal virus according to any of the preceding claims lacking at least one gene which is essential for replication in its natural host or cells or cell lines derived thereof.

8. The recombinant animal virus according to any of the preceding claims, comprising ORI_{S} and/or ORI_{L}, and the packaging (pac) sequences.

9. Use of the recombinant animal virus according to any of the preceding claims as a pharmaceutical or diagnostic agent or a vaccine.

10. Use of the recombinant animal virus according to any one of claims 1 to 8 as a gene targeting vector.

11. Primary cells transduced with the recombinant animal virus according to any one of claims 1 to 8.

12. Packaging cell lines harboring at least one recombinant animal virus according to any one of claims 1 to 8, lacking virus packaging sequences, ORI_{S} and/or ORI_{L}, but provides and complements the required and essential genes removed from the vectors for virus DNA packaging *in trans.*
